(19) Europäisches Patentamt
European Patent Office
Office européen des brevets

(11) EP 3 784 230 B1

(12) **EUROPEAN PATENT SPECIFICATION**

(45) Date of publication and mention of the grant of the patent:
**30.11.2022 Bulletin 2022/48**

(21) Application number: **19782796.7**

(22) Date of filing: **26.04.2019**

(51) International Patent Classification (IPC):
***A61K 31/37*** (2006.01) ***A61P 35/00*** (2006.01)
***A61K 45/06*** (2006.01)

(52) Cooperative Patent Classification (CPC):
**A61K 31/37; A61K 45/06; A61P 35/00**

(86) International application number:
**PCT/TR2019/050284**

(87) International publication number:
**WO 2019/209232 (31.10.2019 Gazette 2019/44)**

(54) **THE USE OF TERPENIC COUMARINE DERIVATIVE MOLECULES IN THE TREATMENT OF CANCER DISEASE**

VERWENDUNG VON TERPENISCHEN CUMARINDERIVATMOLEKÜLEN ZUR BEHANDLUNG VON KREBSERKRANKUNGEN

UTILISATION DE MOLÉCULES DÉRIVÉES DE COUMARINE TERPÉNIQUES DANS LE TRAITEMENT D'UNE MALADIE CANCÉREUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2018 TR 201805902**

(43) Date of publication of application:
**03.03.2021 Bulletin 2021/09**

(73) Proprietors:
- **T.C. Istanbul Medipol Universitesi Istanbul (TR)**
- **T.C. Istanbul Üniversitesi Fatih/Istanbul (TR)**

(72) Inventors:
- **TOSUN, Fatma Istanbul (TR)**
- **MISKI , Mahmud Istanbul (TR)**

(74) Representative: **Simsek, Meliha Merve Incirli Cd. Ulku Sk. No:12, B Blok, Daire:6 34145 Bakirkoy/Istanbul (TR)**

(56) References cited:
**CN-A- 103 585 209 CN-A- 103 585 211**

- **ANNA GLISZCZYNSKA ET AL: "Sesquiterpene coumarins", PHYTOCHEMISTRY REVIEWS, KLUWER ACADEMIC PUBLISHERS, DO, vol. 11, no. 1, 18 November 2011 (2011-11-18), pages 77-96, XP035015555, ISSN: 1572-980X, DOI: 10.1007/S11101-011-9220-6**
- **LI GUANGZHI ET AL: "Sesquiterpene coumarins from seeds ofFerula sinkiangensis", FITOTERAPIA, IDB HOLDING, MILAN, IT, vol. 103, 27 March 2015 (2015-03-27), pages 222-226, XP029187988, ISSN: 0367-326X, DOI: 10.1016/J.FITOTE.2015.03.022**
- **FATMA TOSUN ET AL: "Anatolicin, a Highly Potent and Selective Cytotoxic Sesquiterpene Coumarin from the Root Extract of Heptaptera anatolica", MOLECULES, vol. 24, no. 6, 23 March 2019 (2019-03-23) , page 1153, XP055652508, DOI: 10.3390/molecules24061153**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 784 230 B1

## Description

### Technical Field

The present invention relates to compounds suitable for use in the treatment of cancer disease and to pharmaceutical compositions comprising said compounds as defined in the claims.

### State of the Art

Cancer is a disease wherein the mutation of cells invade the body by uncontrolled proliferation. Cancer has no definitive treatment yet and it is often the results with the death of the patient. In the state of the art, a variety of chemical agents are used for the treatment or for the elimination of symptoms caused by or for slowing the progression of this disease. These agents function through different mechanisms, such as inhibiting cell division, inhibiting various enzymes that provide cell proliferation, inhibiting the formation of vascularity around the tumor, or inhibiting the nutrition of cells. The selectivity of the anti-cancer agents present in the state of the art is quite low, so the agents in question also act on healthy cells in addition to the tumor cells, causing serious side effects of the patients. The lack of definitive treatment of cancer causes the drugs used in the treatment to be less selective and continue to be used despite their serious side effects.

ANNA GLISZCZYNSKA ET AL: "Sesquiterpene coumarins", PHYTOCHEMISTRY REVIEWS, vol. 11, no. 1, 18 November 2011 (2011-11-18), pages 77-96, discloses the anticancer activity of colladonin against colorectal cancer cells.

LI GUANGZHI ET AL: "Sesquiterpene coumarins from seeds ofFerula sinkiangensis", FITOTERAPIA, vol. 103, 27 March 2015 (2015-03-27), pages 222-226, discloses the anticancer activity of colladonin against K562 myelogenous leukemia and AGS adenocarcinoma cancer cell lines.

CN 103 585 209 and CN 103 585 211 disclose the anticancer activity of Dorema Ammoniacum resin extract mainly comprising sec-butyl-1-acrylic disulphide, diallyl disulfide, badrakemin, badrakemin acetate, badrakemone, feshurin and colladonin against liver cancer and leukemia.

### Brief Description of the Invention

When the state of the art is examined it is seen that new anti-cancer agents with high selectivity, low side effects and high efficacy in comparison to the agents present in the prior art are needed.

The inventors have found that terpenic coumarin-derived compounds are effective on cancer cells.

### Detailed Description of the Invention

The present invention relates to 14'-acetoxybadrakemin, 14'-acetoxycolladonin or 14'-hydroxycolladonin or the salts, hydrates, solvates, polymorphs, stereoisomers, optical isomers, geometric isomers, enantiomers, diastereoisomers and/or combinations thereof for use in the treatment of cancer.

In one embodiment, 14'-acetoxybadrakemin, 14'-acetoxycolladonin or 14'-hydroxycolladonin are for use in treatment of B cell lymphoma, T cell lymphoma, mycosis fungoides, Hodgkin's disease, bladder cancer, brain cancer, nervous system cancer, head and neck cancer, squamous cell carcinomas of the head and neck, kidney cancer, small cell lung cancer and non-small cell lung cancer, neuroblastoma / glioblastoma, ovarian cancer, pancreatic cancer, prostate cancer, skin cancer, liver cancer, melanoma, squamous cell carcinoma of the mouth, throat, esophagus, colon cancer, cervical cancer, cervical carcinoma, breast cancer and epithelial cancer, kidney cancer, genitourinary cancer, pulmonary cancer, esophageal carcinoma, head and neck carcinoma, colorectal cancer, hematopoietic cancers; testicular cancer.

In a further aspect, the present invention relates to a pharmaceutical composition comprising 14'-acetoxybadrakemin, 14'-acetoxycolladonin or 14'-hydroxycolladonin or pharmaceutically acceptable salts, stereoisomers, optical isomers, enantiomers, diastereoisomers, and/or combinations thereof.

The pharmaceutical composition may further comprise at least one or more excipients in addition to the active ingredient according to the invention.

In one aspect, the pharmaceutical composition further comprises at least one other active ingredient.

In another aspect, the other active ingredient is selected from a group comprising anticancer agents, nucleoside analogues, antifolates, antimetabolites, topoisomerase I inhibitors, anthracyclines, podophyllotoxins, taxanes, vinca alkaloids, alkylating agents, platinum compounds, antihormones, radiopharmaceuticals, monoclonal antibodies, tyrosine kinase inhibitors, mammalian target (mTOR) inhibitors, retinoids, immunoregulatory agents and histoneacetylase inhibitors.

In another aspect the present invention relates to colladonin or badrakemin for use in treatment of kidney cancer.

In a non-claimed aspect, the present disclosure relates to compounds of Formula I and / or Formula II;

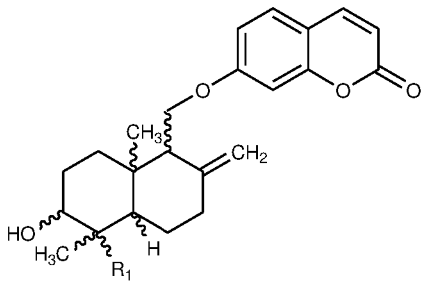

*Formula I*

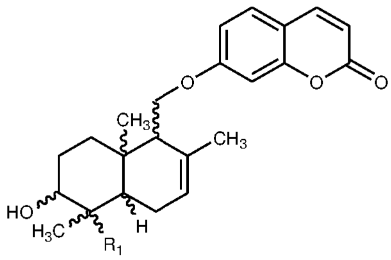

*Formula II*

wherein;

R1 is selected from

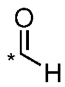 or 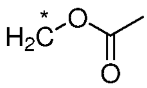 or 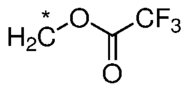

or $\overset{*}{C}H_3$ or $CF_3$ or their salts, hydrates, solvates, polymorphs, stereoisomers, optical isomers, geometric isomers, enantiomers, diastereomers, and/or combinations thereof for the manufacture of a medicament for use in the treatment of cancer.

In other words; in one non-claimed aspect, the present disclosure relates to molecules of Formula I and/or Formula II

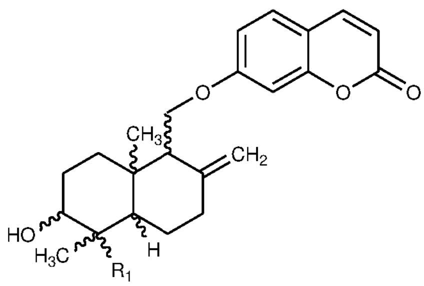

*Formula I*

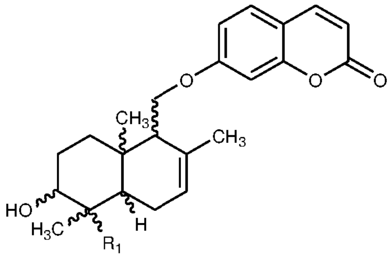

*Formula II*

wherein;
R1 is selected from

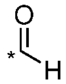 or 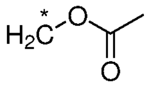 or 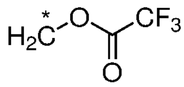

or 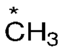 or $CF_3$ or their salts, hydrates, solvates, polymorphs, stereoisomers, optical isomers, geometric isomers, enantiomers, diastereomers and / or combinations thereof for use in the treatment of cancer.

The sign "*" shows the point where said R1 group is attached to the structures, which indicates that the group is bonded to the major structures indicated by Formula I and Formula II by a single bond from the point at which it is marked with * sign.

The wavy bond shown with the shape "" used on the structure showing Formula I and Formula II indicates that the bond at that location can be located such that it is directed into the page or out of the page.

It is seen from the drawings that there are many stereocenters on the molecules represented by Formula I and Formula II . The above-described linkages indicate that the groups indicated by the wavy bond at the said stereocenters may be above or below the page .In this context, all different optical isomers resulting from this disclosure are within the scope of this disclosure, although not explicitly shown herein.

In the molecules shown with Formula I and Formula II, the groups that are attached to the molecule with wavy bonds can be directed into the page or out of the page independent from one another. In other words, while one of the groups is directed towards underneath of/into the page surface the other one can be directed towards out of the page surface or all of the groups present on the molecule can be directed towards underneath/into the page surface or out of the page surface or while 1 or 2 or 3 or 4 or 5 of the groups shown with a wavy bond are towards underneath/into the surface of the page 1 or 2 or 3 or 4 or 5 of the groups shown with a wavy bond can be towards out of the page surface. All the different isomers of Formula I and Formula II, which will result from such different combinations, are disclosed herein.

The molecules according to present invention can be rich in one or more diastereomers. For example, the molecules according to the invention can have at least 30% diastereomeric excess (de), 40% de, 50% de, 60% de, 70% de, 80% de, 90% de, or 95% de.

In order to provide a better understanding of the non-claimed aspects, specific examples of the molecules represented by Formula I and Formula II are shown in the following table;

Table 1: Specific examples of parent molecules shown with Formula I and Formula II.

| Number | Structure | Number | Structure |
|---|---|---|---|
| Formula 1.1 | 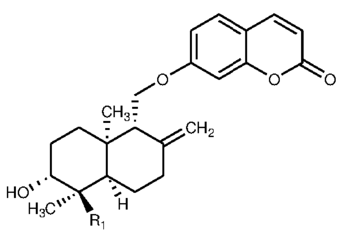 | Formula II. 1 | 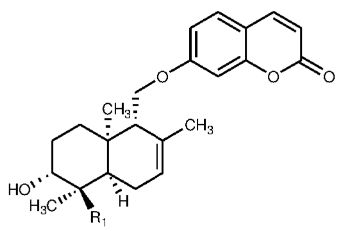 |

(continued)

| Number | Structure | Number | Structure |
|---|---|---|---|
| Formula 1.2 | 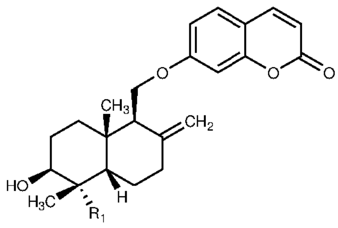 | Formula II.2 | 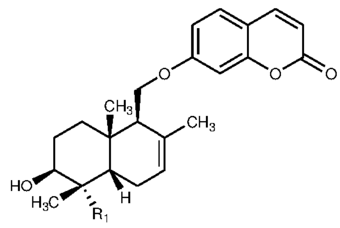 |
| Formula 1.3 | 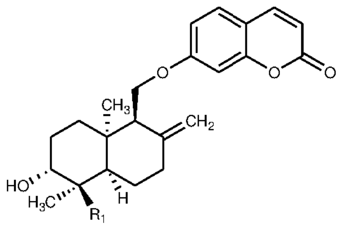 | Formula II.3 | 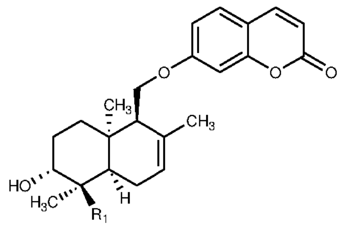 |
| Formula 1.4 | 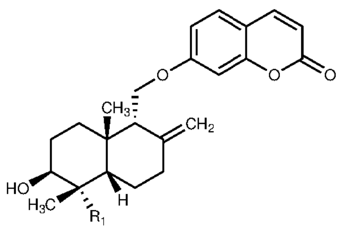 | Formula II.4 | 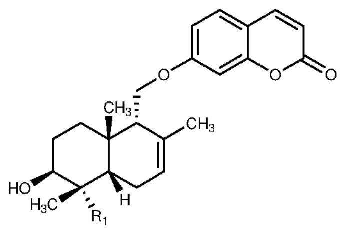 |
| Formula 1.5 | 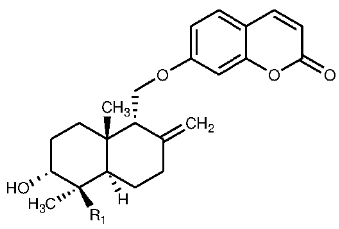 | Formula II.5 | 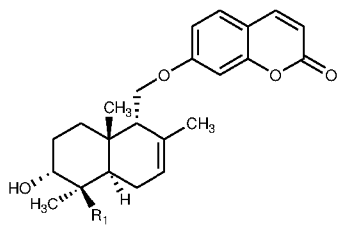 |
| Formula 1.6 | 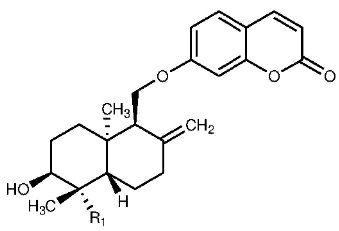 | Formula II.6 | 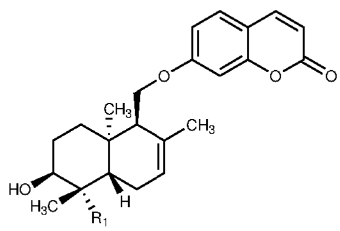 |
| Formula 1.7 | 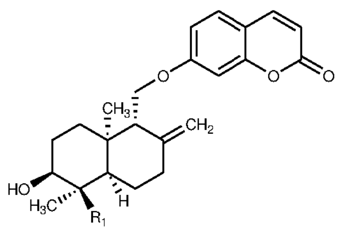 | Formula II.7 | 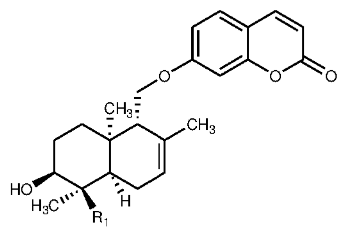 |

(continued)

| Number | Structure | Number | Structure |
|---|---|---|---|
| Formula 1.8 | 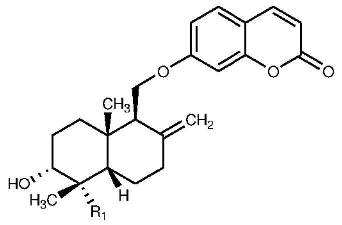 | Formula II.8 | 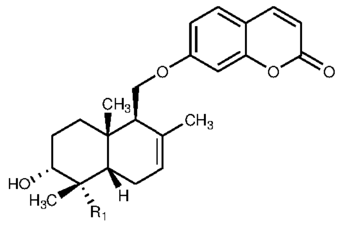 |
| Formula 1.9 | 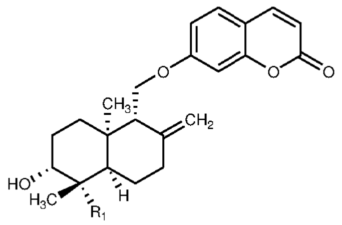 | Formula II.9 | 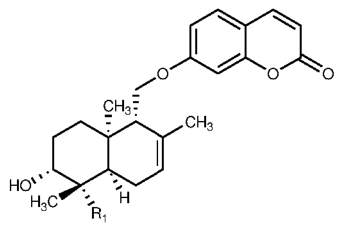 |
| Formula 1.10 | 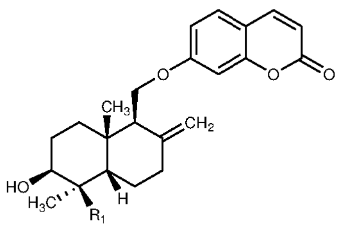 | Formula II.10 | 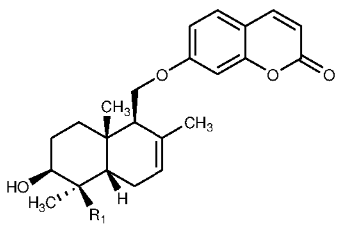 |
| Formula 1.11 | 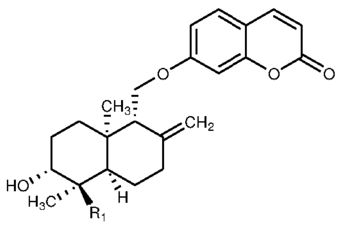 | Formula II.11 | 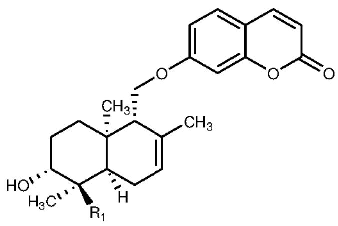 |
| Formula 1.12 | 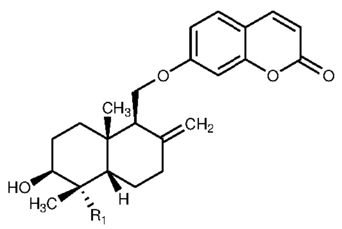 | Formula II.12 | 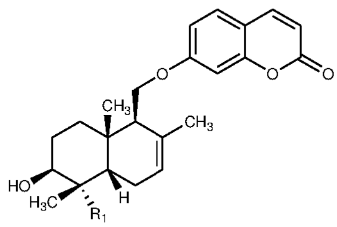 |
| Formula 1.13 | 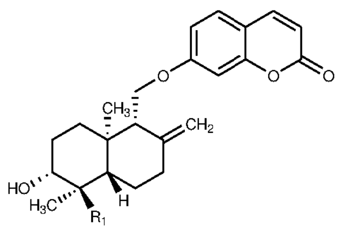 | Formula II.13 | 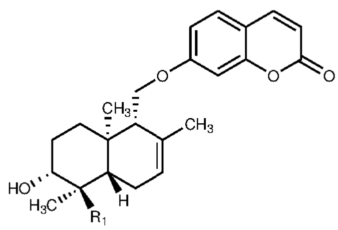 |

(continued)

| Number | Structure | Number | Structure |
|---|---|---|---|
| Formula 1.14 | 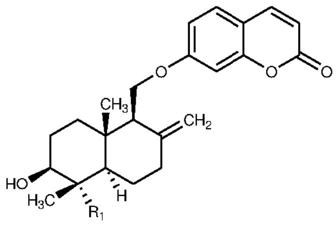 | Formula II.14 | 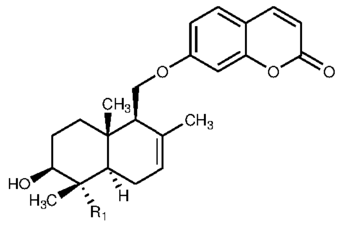 |
| Formula 1.15 | 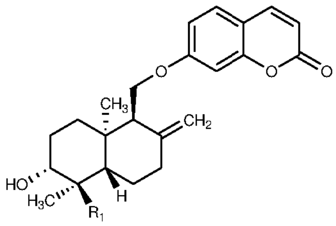 | Formula II.15 | 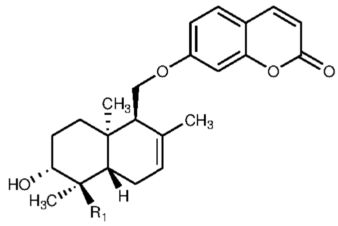 |
| Formula 1.16 | 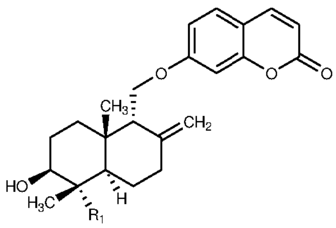 | Formula II.16 | 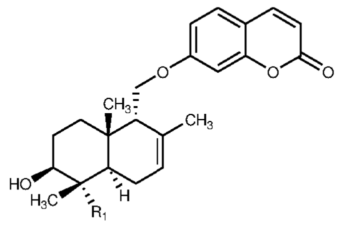 |
| Formula 1.17 | 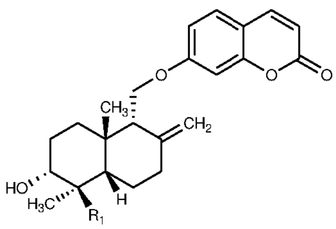 | Formula II.17 | 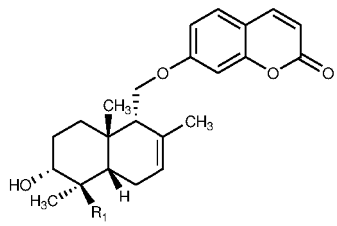 |
| Formula 1.18 | 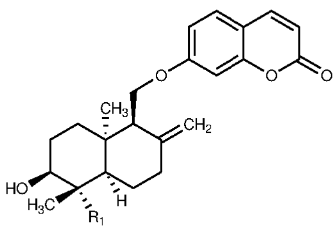 | Formula II. 18 | 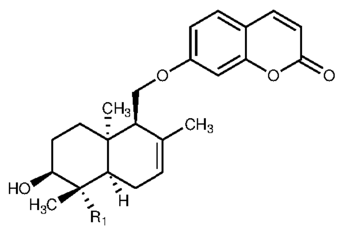 |
| Formula 1.19 | 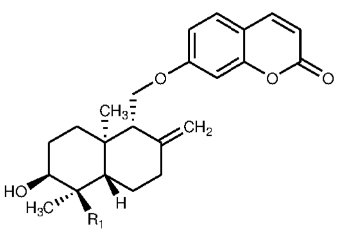 | Formula II.19 | 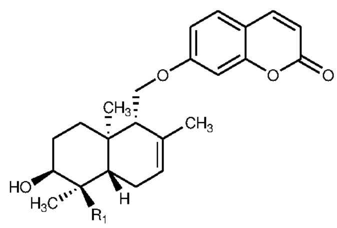 |

(continued)

| Number | Structure | Number | Structure |
|---|---|---|---|
| Formula 1.20 | 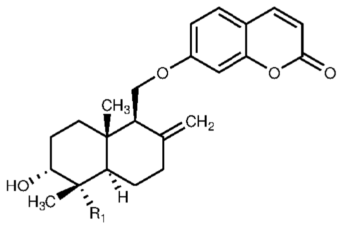 | Formula II.20 | 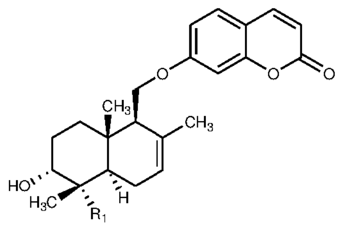 |
| Formula 1.21 | 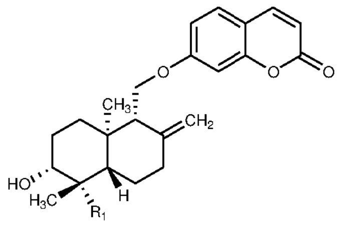 | Formula II.21 | 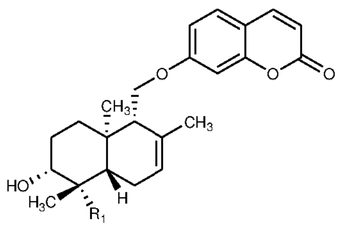 |
| Formula 1.22 | 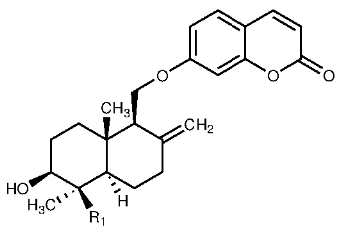 | Formula II.22 | 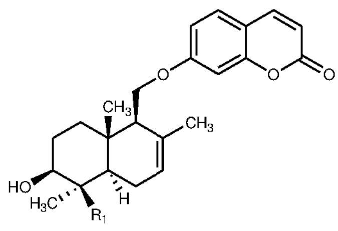 |
| Formula 1.23 | 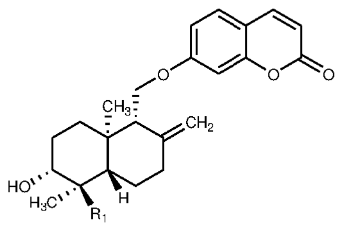 | Formula II.23 | 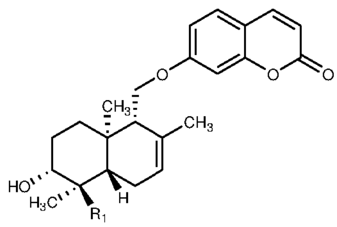 |
| Formula 1.24 | 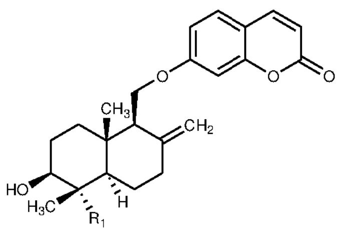 | Formula II.24 | 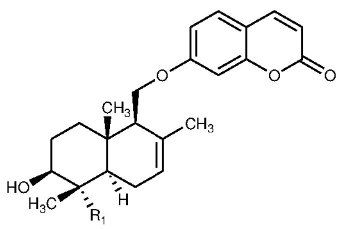 |
| Formula 1.25 | 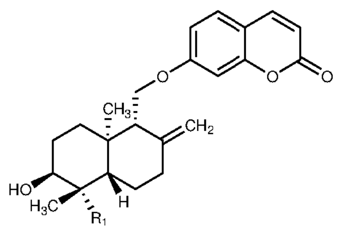 | Formula II.25 | 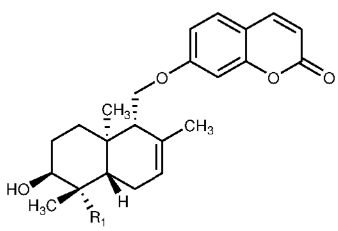 |

In the specific examples of Formula I and Formula II given in the above table, the R1 group is, as previously described, selected from

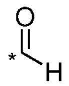 or 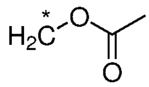

or

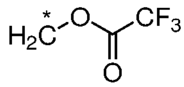

or $\overset{*}{C}H_3$ or $\overset{*}{C}F_3$ .

The molecules of Formula I and Formula II can be prepared and purified using methods known in the art.

In another non-claimed aspect, the present disclosure relates to Formula I and / or Formula II, or pharmaceutically acceptable salts, prodrugs, stereoisomers, optical isomers, enantiomers, diastereomers and / or combinations thereof, for use in the treatment or prevention of cancer.

In another non-claimed aspect, the present disclosure relates to Formula I and / or Formula II or pharmaceutically acceptable salts, prodrugs, stereoisomers, optical isomers, enantiomers, diastereomers, and / or combinations thereof, for use in the manufacture of a medicament for the treatment or prevention of cancer.

As used herein, "treatment" or "to treat" refers to the prevention, reduction, alleviation, amelioration or blocking of at least one symptom that characterizes a pathological disorder in a subject that is threatened by a disorder or has a condition.

The terms "cancer" and "cancerous" as used herein refer to malignant tumors or describe any physiological condition characterized by uncontrolled cell growth.

The following is a non-limiting list of different types of cancer: carcinomas, solid carcinomas, squamous cell carcinomas, adenocarcinomas, sarcomas, gliomas, high-grade gliomas, blastomas, neuroblastomas, plasmacytomas, histiocytomas, melanomas, adenomas, hypoxic tumors, myeloma, metastatic cancers or cancers in general. Specific examples of cancer that can be used in the treatment of the compounds described herein include B-cell lymphoma, T-cell lymphoma, mycosis fungoides, Hodgkin's disease, bladder cancer, brain cancer, nervous system cancer, head and neck cancer, squamous cell carcinomas of the head and neck, kidney cancer, small cell carcinoma of the lung, non-small cell lung cancer, lung cancers, neuroblastoma / glioblastoma, ovarian cancer, pancreatic cancer, prostate cancer, skin cancer, liver cancer, melanoma, squamous cell carcinoma of the mouth, throat, esophagus, colon cancer, cervical cancer , cervical carcinoma, breast cancer and epithelial cancer, kidney cancer, genitourinary cancer, pulmonary cancer, esophageal carcinoma, head and neck carcinoma, large intestine cancer, hemotopoietic cancers; testicular cancer; colon and rectal cancers, prostate cancer or pancreatic cancer. Molecules shown with Formula I and Formula II can be used in the treatment of one of, some of or all of the cancer types or for elimination of the symptoms thereof or for preventing emergence of said symptoms.

The molecules disclosed herein can also be used for the treatment of pre-cancerous disorders such as cervical and anal dysplasias, other dysplasias, severe dysplasias, hyperplasia, non-typical hyperplasia and neoplasia.

As disclosed herein, the molecules of the invention find use in the treatment of cancer and/or protection from cancer disease, as defined in the claims. For use in this manner, the molecules of the present invention will preferably be administered in the form of a pharmaceutical composition.

One non-claimed aspect, the disclosure relates to pharmaceutical compositions comprising Formula I and/or Formula II or pharmaceutically acceptable salts, prodrugs, stereoisomers, optical isomers, enantiomers, diastereomers, and/or combinations thereof.

In another non-claimed aspect, the disclosure relates to pharmaceutical compositions suitable for use in the treatment of cancer, comprising Formula I and/or Formula II, or pharmaceutically acceptable salts, prodrugs, stereoisomers, optical isomers, enantiomers, diastereomers, and/or combinations thereof as active ingredients. The pharmaceutical compositions comprise at least one or more excipients in addition to the active ingredients according to the invention.

In another non-claimed aspect of the disclosure the pharmaceutical composition comprising Formula I and/or Formula II or pharmaceutically acceptable salts, prodrugs, stereoisomers, optical isomers, enantiomers, diastereomers, and/or combinations thereof as active ingredient may further comprise at least one other active ingredient.

Said other active ingredient can be selected from, but not limited to, a subgroup comprising anticancer agents, nucleoside analogs, antifolates, antimetabolites, topoisomerase I inhibitors, anthracyclines, podophyllotoxins, taxanes, vinca alkaloids, alkylating agents, platinum compounds, antihormones, radiopharmaceuticals, monoclonal antibodies,

tyrosine kinase inhibitors, mammalian target of rapamycin ( mTOR) inhibitors, retinoids, immunoregulatory agents, histoneacetylase inhibitors and other agents.

Anticancer agents can be selected from a group comprising, docetaxel, gemcitabine, imatinib, 5-fluorouracil, 9-aminocamptothecin, amine-modified geldanamycin, doxorubicin, paclitaxel, procarbazine, hydroxyurea, meso e-chlorine, cisplatin and radionuclides (eg, 1-131, Y-90, In- 111 and Tc-99m).

The nucleoside analogs can be selected from a group comprising, but not limited to, azacitidine, cladribine, clofarabine, cytarabine, decitabine, floxuridine, fludarabine, 5-fluorouracil (5-FU), gemcitabine, mercaptopurine, nelarabine, pentostatin, thioguanine, trifluridine and tipiracil.

Antifolates can be selected from a group comprising, but not limited to, methotrexate, pemetrexed, pralatrexed and raltitrexed.

Antimetabolites can be selected from a group comprising, but not limited to, hydroxycarbamide.

Topoisomerase I inhibitors can be selected from a group comprising, but not limited to, irinotecan and topotecan.

Anthracyclines can be selected from a group comprising, but not limited to, daunorubicin, doxorubicin, epirubicin, idarubicin, mitoxantrone and valrubicin.

Podophilotoxins can be selected from a group comprising, but not limited to, etoposide and teniposide.

Taxanes can be selected from a group comprising, but not limited to, cabasitaxel, docetaxel and paclitaxel.

Vinca alkaloids can be selected from the group comprising, but not limited to, vinblastine, vincristine, vindesin, vinflunine and vinorelbine.

Alkylating agents can be selected from the group comprising, but not limited to, bendamustine, chlorambucil, dacarbazine, melphalan, streptozotocin and trabestedin.

Antihormone compounds can be selected from a group comprising, but not limited to, abiraterone, bicalutamide, ciproteron, degarelic, exemestane, fulvestrant, goserelin, histrelin, leuprolide, mifepristone and triptorel.

Tyrosine kinase inhibitors can be selected from a group comprising, but not limited to, afatinib, axitinib, bosutinib, cobimetinib, crizotinib, dasatinib, erlotinib, gefitinib, imatinib, lapatinib, nilotinib, osimertinib, pazopanib, rucolinolinib, sunitinib and vandetanib.

The mammalian target (mTOR) inhibitors of rapamycin can be selected from a group comprising but not limited to everolimus and temsirolimus.

Retinoids can be selected from a group comprising, but not limited to, alytretinoin, bexarotene, isotretinoin, tamibarotene and tretinoin.

Immune system regulators may be selected from a group including, but not limited to, lenalidomide, pomalidomide and thalidomide.

Histone deacetylase inhibitors may be selected from a group comprising, but not limited to, belinostat, panobinostat, valproate and vorinostate.

Other substances can be selected from a group comprising, but not limited to, anagrelide, seritinib, dabrafenib, idelalisib, ibrutinib, palbociclib, vemurafenib, bleomycin, bortezomib, dactinomycin, eribulin, estramustine, ixabepilone, mitomycin, procarbazine, alectinib, flucimesterone, iobenguane, imiguimod, interferon, ixazomib, lanreotide, lentinan, octreotide, omacetaxine, tegafur, gimerazil, oteracil, uracil, combrestatin and chloroquine.

The composition comprising the molecules of the invention can be in any suitable form based on the preferred method of administering the composition to a patient. The composition comprising the molecules of the present invention can be formulated for example for oral administration in the form of liquid dispersions or aqueous or oily suspensions, or in parenteral form, for example, in forms suitable for subcutaneous, intravenous, intramuscular, intraperitoneal, intradermal, transdermal or other infusion techniques. The composition comprising the molecules of the invention may also be formulated in the form of a spray tube for administration through inhalation or as a solution for administration by inhalation device or nebulizer. The molecules of the present invention are preferably administered to a patient by transdermal, subcutaneous, intranasal, intravenous, intramuscular, intratumorally or by inhalation. In any case, the most suitable route for administration can be determined based on the molecules of the present invention , the nature and severity of the disease and the patient’s physical condition.

In the context of this specification, it is intended that the expression "comprise" also confers the expression "include" .

Where technically feasible, the embodiments of the invention can be combined. Embodiments are described here to include specific features / elements.

The disclosure also encompasses essentially other applications comprising or consisting of said features / elements.

The specific and clearly described embodiments herein may form the basis of a disclaimer, either alone or in combination with one or more other embodiments.

The invention will now be described by way of example only with reference to the following examples, which are intended to be exemplary only and are not to be construed in any way as limiting the scope of the invention.

**EXAMPLES:**

The inventors have determined the cytotoxic activity (IC $_{50}$ values) of the compounds according to the invention together with various agents present in the prior art on cell lines of different types of cancer. The results of these tests are presented in the table below (Table 2).

Table 2: IC $_{50}$ data demonstrating the efficacy of molecules and reference molecules according to the invention on various cancer cell lines

| | **Cytotoxic Activity** ( IC $_{50}$ ) **(uM)** | | | | | |
|---|---|---|---|---|---|---|
| | COLO205 | KM12 | A498 | U031 | A673 | TC32 |
| 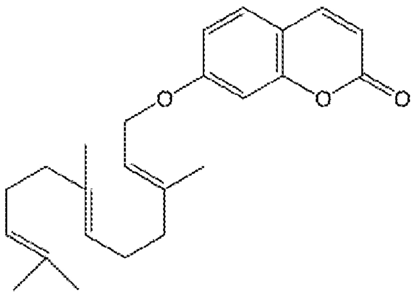 **Umbelliprenin** (REFERENCE MOLECULE I) | > 50 | > 50 | > 50 | 1.8 | > 50 | > 50 |
| 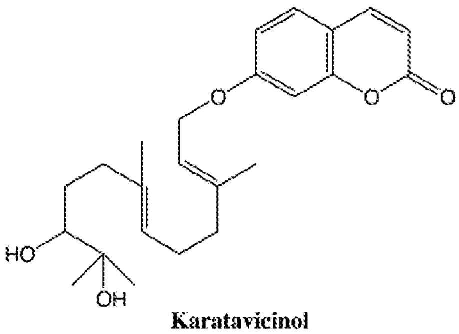 **Karatavicinol** (REFERENCE MOLECULE II) | > 50 | > 50 | > 50 | 7.6 | > 50 | > 50 |
| 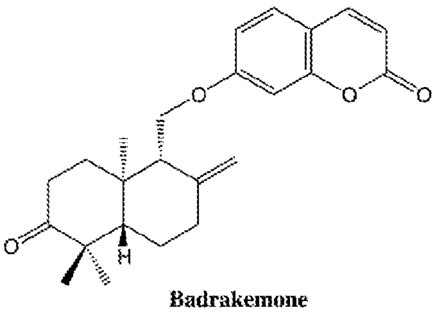 **Badrakemone** (REFERENCE MOLECULE III) | > 50 | > 50 | > 50 | 11 | > 50 | > 50 |

(continued)

| | **Cytotoxic Activity** ( IC $_{50}$ ) **(uM)** | | | | | |
|---|---|---|---|---|---|---|
| | COLO205 | KM12 | A498 | U031 | A673 | TC32 |
| 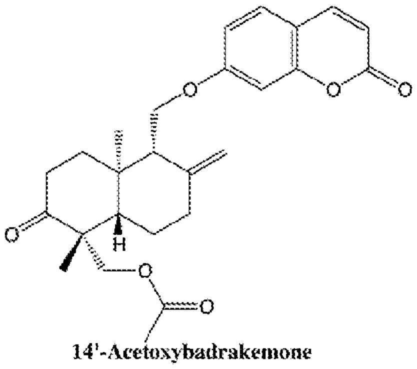 14'-Acetoxybadrakemone (REFERENCE MOLECULE IV) | > 50 | > 50 | > 50 | > 50 | > 50 | > 50 |
| 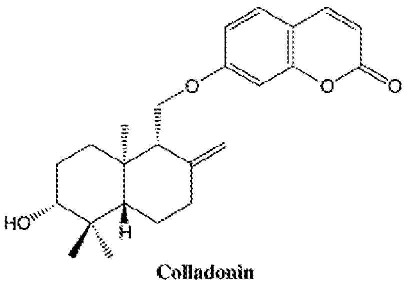 Colladonin (EXAMPLE MOLECULE I) | 19 | 2.5 | 21 | 0.75 | > 50 | 45 |
| 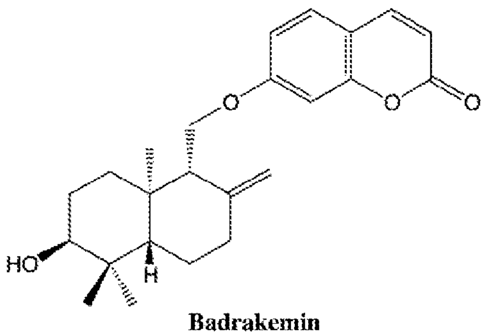 Badrakemin (EXAMPLE MOLECULE II) | > 50 | 9.1 | 20 | 0.38 | > 50 | > 50 |
| 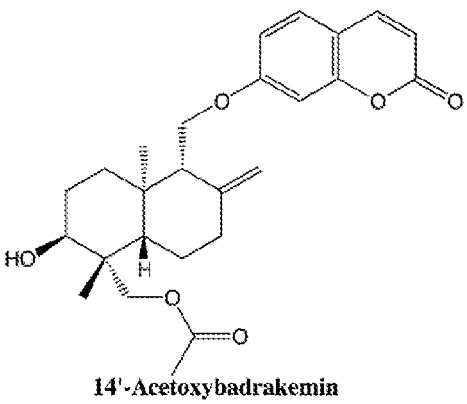 14'-Acetoxybadrakemin (EXAMPLE MOLECULE III) | > 50 | > 50 | > 50 | 0,017 | > 50 | > 50 |

(continued)

| | Cytotoxic Activity ( $IC_{50}$ ) (uM) | | | | | |
|---|---|---|---|---|---|---|
| | COLO205 | KM12 | A498 | U031 | A673 | TC32 |
| 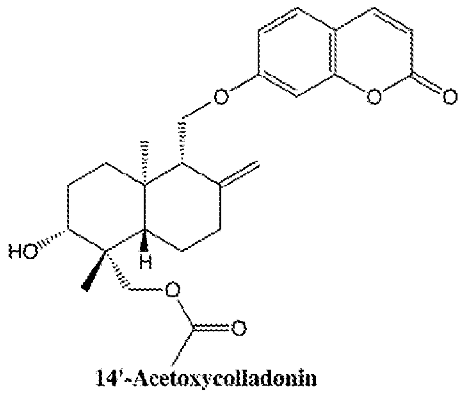 14'-Acetoxycolladonin (EXAMPLE MOLECULE IV) | > 50 | > 50 | > 50 | 0.37 | > 50 | > 50 |
| 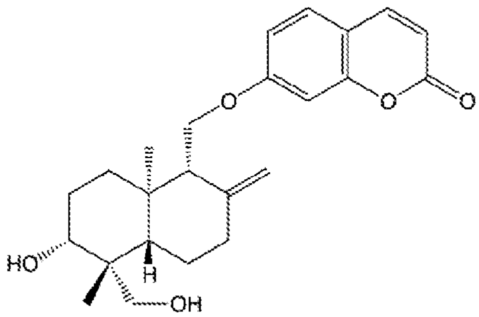 14'-Hydroxycolladonin (REFERENCE MOLECULE V) | > 50 | > 50 | > 50 | > 50 | > 50 | > 50 |
| 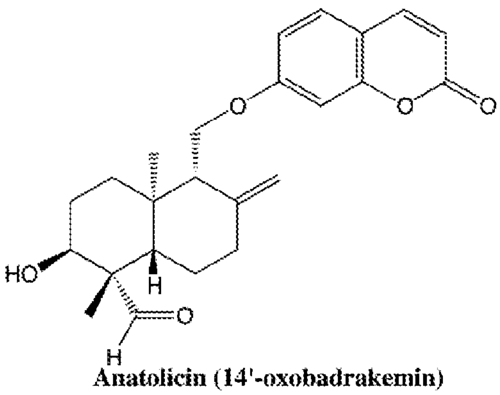 Anatolicin (14'-oxobadrakemin) (EXAMPLE MOLECULE V) | > 50 | > 50 | > 50 | 0,024 | > 50 | > 50 |

When the data in the above table are analyzed, it can be seen that some of the molecules according to the invention are effective also in cell lines where the reference molecules do not have significant effects (e.g. COLO205, KM12, A498, A673, TC32). Furthermore, the molecules according to the invention appear to be more effective than the reference molecules in the UO31 cell line, where the reference molecules show activity. In this context, the molecules according to the invention surpass the state of the art.

The UO31 cell line is known to be the kidney cancer cell line. Taxol, vinblastine and vincristine anti-cancer agents are used against the kidney cancer in the state of the art. For UO31 cell line; The $IC_{50}$ value of taxol from these anticancer agents is greater than 50 uM, and the $IC_{50}$ value of vinblastine and vincristine is greater than 100 uM (Ferguson R.).E. et. al., Int.J. Cancer 2005 , 115, 155-163).Thus, the molecules according to the invention show superior efficacy than the taxol, vinblastine and vincristine which are the molecules used in the treatment of cancer in the prior art.

**Claims**

1. 14'-Acetoxybadrakemin, 14'-Acetoxycolladonin or 14'-Hydroxycolladonin or the salts, hydrates, solvates, polymorphs, stereoisomers, optical isomers, geometric isomers, enantiomers, diastereoisomers and / or combinations thereof for use in the treatment of cancer.

2. A molecule according to claim 1, for use in treatment of B cell lymphoma, T cell lymphoma, mycosis fungoides, Hodgkin's disease, bladder cancer, brain cancer, nervous system cancer, head and neck cancer, squamous cell carcinomas of the head and neck, kidney cancer, small cell lung cancer and non-small cell lung cancer, neuroblastoma / glioblastoma, ovarian cancer, pancreatic cancer, prostate cancer, skin cancer, liver cancer, melanoma, squamous cell carcinoma of the mouth, throat, esophagus, colon cancer, cervical cancer, cervical carcinoma, breast cancer and epithelial cancer, kidney cancer, genitourinary cancer, pulmonary cancer, esophageal carcinoma, head and neck carcinoma, colorectal cancer, hematopoietic cancers; testicular cancer.

3. Colladonin or badrakemin for use in treatment of kidney cancer.

4. A pharmaceutical composition comprising 14'-Acetoxybadrakemin, 14'-Acetoxycolladonin or 14'-Hydroxycolladonin or pharmaceutically acceptable salts, stereoisomers, optical isomers, enantiomers, diastereoisomers and / or combinations thereof.

5. The pharmaceutical composition according to claim 4 further comprising at least one or more excipients in addition to the active ingredient according to the invention.

6. The pharmaceutical composition according to claims 4 and 5 further comprising at least one other active ingredient.

7. A pharmaceutical composition according to claim 6, wherein other active ingredient is selected from a group comprising anticancer agents, nucleoside analogues, antifolates, antimetabolites, topoisomerase I inhibitors, anthracyclines, podophyllotoxins, taxanes, vinca alkaloids, alkylating agents, platinum compounds, antihormones, radiopharmaceuticals, monoclonal antibodies, tyrosine kinase inhibitors, mammalian target (mTOR) inhibitors, retinoids, immunoregulatory agents, and histoneacetylase inhibitors.

**Patentansprüche**

1. 14'-Acetoxybadrakemin, 14'-Acetoxycolladonin oder 14'-Hydroxycolladonin oder die Salze, Hydrate, Lösungsmittel, Polymorphe, Stereoisomere, optische Isomere, geometrische Isomere, Enantiomere, Diastereoisomere und/oder Kombinationen davon zur Verwendung bei der Behandlung von Krebs.

2. Molekül nach Anspruch 1 zur Verwendung bei der Behandlung von B-Zell-Lymphom, T-Zell-Lymphom, Mycosis fungoides, Morbus Hodgkin, Blasenkrebs, Hirnkrebs, Krebs des Nervensystems, Kopf- und Halskrebs, Plattenepithelkarzinomen des Kopfes und Halses, Nierenkrebs, kleinzelligem Lungenkrebs und nicht-kleinzelligem Lungenkrebs, Neuroblastom / Glioblastom, Eierstockkrebs, Bauchspeicheldrüsenkrebs, Prostatakrebs, Hautkrebs, Leberkrebs, Melanom, Plattenepithelkarzinom des Mundes, des Rachens, des Ösophagus, Dickdarmkrebs, Gebärmutterhalskrebs, Gebärmutterhalskarzinom, Brustkrebs und Epithelkrebs, Nierenkrebs, Krebs der Urogenitalorgane, Lungenkrebs, Ösophaguskarzinom, Kopf-Hals-Karzinom, Dickdarmkrebs, Blutkrebs, Hodenkrebs.

3. Colladonin oder Badrakemin zur Verwendung bei der Behandlung von Nierenkrebs.

4. Pharmazeutische Zusammensetzung, umfassend 14'-Acetoxybadrakemin, 14'-Acetoxycolladonm oder 14'-Hydroxycolladonin oder pharmazeutisch akzeptable Salze, Stereoisomere, optische Isomere, Enantiomere, Diastereoisomere und/oder Kombinationen davon.

5. Pharmazeutische Zusammensetzung nach Anspruch 4, umfassend ferner mindestens einen oder mehrere Hilfsstoffe zusätzlich zu dem erfindungsgemäßen Wirkstoff.

6. Pharmazeutische Zusammensetzung nach den Ansprüchen 4 und 5, umfassend ferner mindestens einen weiteren Wirkstoff.

**7.** Pharmazeutische Zusammensetzung nach Anspruch 6, wobei der weitere Wirkstoff aus einer Gruppe ausgewählt ist, die Antikrebsmittel, Nukleosidanaloga, Antifolate, Antimetabolite, Topoisomerase-I-Inhibitoren, Anthracycline, Podophyllotoxine, Taxane, Vinca-Alkaloide, Alkylierungsmittel, Platinverbindungen, Antihormone, Radiopharmaka, monoklonale Antikörper, Tyrosinkinase-Inhibitoren, Mammalian Target (mTOR) Inhibitoren, Retinoide, immunregulatorische Mittel und Histonacetylase-Inhibitoren umfasst.

## Revendications

**1.** 14'-Acétoxybadrakemine, 14'-Acétoxycolladonin ou 14'-Hydroxycolladonin ou les sels, hydrates, solvates, polymorphes, stéréoisomères, isomères optiques, isomères géométriques, énantiomères, diastéréoisomères et/ou leurs combinaisons pour une utilisation dans le traitement du cancer.

**2.** Molécule selon la revendication 1, pour une utilisation dans le traitement du lymphome à cellules B, du lymphome à cellules T, du mycosis fongoïde, de la maladie de Hodgkin, du cancer de la vessie, du cancer du cerveau, du cancer du système nerveux, du cancer de la tête et du cou, des carcinomes spinocellulaires de la tête et du cou, du cancer du rein, du cancer du poumon à petites cellules et du cancer du poumon non à petites cellules, du neuroblastome/glioblastome, du cancer de l'ovaire, du cancer du pancréas, du cancer de la prostate, du cancer de la peau, du cancer du foie, mélanome, de carcinome spinocellulaire de la bouche, de la gorge, de l'oesophage ; du cancer du côlon, du cancer du col de l'utérus, de carcinome du col de l'utérus, du cancer du sein et cancer épithélial, du cancer du rein, du cancer génito-urinaire, du cancer pulmonaire, de carcinome de l'œsophage, carcinome de la tête et du cou, du cancer colorectal, des cancers hématopoïétique, du cancer des testicules.

**3.** Colladonine ou badrakemine pour une utilisation dans le traitement du cancer du rein.

**4.** Composition pharmaceutique comprenant de la 14'-Acétoxybadrakemine, de la 14'-Acétoxycolladonine ou de la 14'-Hydroxycolladonine ou des sels, stéréoisomères, isomères optiques, énantiomères, diastéréoisomères et/ou combinaisons de ceux-ci pharmaceutiquement acceptables.

**5.** Composition pharmaceutique selon la revendication 4 comprenant en outre au moins un ou plusieurs excipients en plus du principe actif selon l'invention.

**6.** Composition pharmaceutique selon les revendications 4 et 5 comprenant en outre au moins un autre principe actif.

**7.** Composition pharmaceutique selon la revendication 6, dans laquelle l'autre ingrédient actif est choisi dans un groupe comprenant des agents anticancéreux, des analogues de nucléosides, des antifolates, des antimétabolites, des inhibiteurs de la topoisomérase I, des anthracyclines, des podophyllotoxines, des taxanes, les alcaloïdes de la pervenche, les agents alkylants, les composés du platine, les antihormones, les produits radiopharmaceutiques, les anticorps monoclonaux, les inhibiteurs de tyrosine kinase, les inhibiteurs de la cible mammalienne (mTOR), les rétinoïdes, les agents immunorégulateurs et les inhibiteurs d'histone-acétylase.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103585209
- CN 103585211


### Non-patent literature cited in the description


- **ANNA GLISZCZYNSKA et al.** Sesquiterpene coumarins. *PHYTOCHEMISTRY REVIEWS,* 18 November 2011, vol. 11 (1), 77-96
- **LI GUANGZHI et al.** Sesquiterpene coumarins from seeds ofFerula sinkiangensis. *FITOTERAPIA,* 27 March 2015, vol. 103, 222-226
- **FERGUSON R.).E.** *Int.J. Cancer,* 2005, vol. 115, 155-163